# EUROPEAN PATENT APPLICATION

(11) **EP 4 011 352 A1**
(43) Date of publication of application: **15.06.2022**
(21) Application number: 20212728.8
(22) Date of filing: 09.12.2020
(51) Int. Cl.: A61K 8/34, A61K 8/44, A61K 8/60, A61Q 5/00, A61Q 17/04, A61Q 19/00, A61K 8/365, A61K 8/42

(54) **NEW COSMETICS SOLVENTS BASED ON THREE DIFFERENT COMPONENTS**

(71) Applicant: Beiersdorf AG, 20253 Hamburg (DE)
(72) Inventor: Piacentini, Adalberto, 22529 Hamburg (DE); Köhler, Manuela, 22527 Hamburg (DE); Kühnl,, Jochen, 20249 Hamburg (DE); Bernau, Mareike, 22087 Hamburg (DE); Ferraro, Claudio, 1007 Lausanne (CH)
(74) Representative: Beiersdorf AG

(57) **Abstract**

The invention is an eutectic solvent which is formed from the mixture of three different components selected from the group consisting of Betaine, Glycerol, Urea, Citric Acid, Malic Acid, Glucose, Fructose, Sorbitol, Sucrose, Xylitol, Proline and Choline Chloride in precise molar ratio. The new inventive eutectic solvents allows an improved incorporation of active ingredients into cosmetic compositions.

## Description

The invention is an eutectic solvent which is formed from the mixture of three different components selected from the group consisting of Betaine, Glycerol, Urea, Citric Acid, Malic Acid, Glucose, Fructose, Sorbitol, Sucrose, Xylitol, Proline and Choline Chloride in precise molar ratio. The new inventive eutectic solvents allows an improved incorporation of active ingredients into cosmetic compositions.

Natural deep eutectic solvents (NADES) are bio-based ionic liquids and deep eutectic solvents which are composed of two or more compounds that are generally plant based primary metabolites, i.e. organic acids, sugars, alcohols, amines and amino acids. Ionic liquids are salts, liquid at room temperature, characterized by ionic bonds which have at least one large organic ion and a cation with a low degree of symmetry. The positive and negatively charged ions in the liquid are thus kept far apart so that they reduce the attractive forces between them and hinder crystallization, lowering the melting point and resulting in a completely ionic liquid at room temperature. Deep eutectic solvents (DES) are mixtures of solid compounds that form liquids due to a large depression of the melting point; the driving force in the case of DES is the extensive hydrogen bonding between components forming a non-aqueous liquid at room temperature. Work done by Choi, Spronsen *et al.* showed that water can be present as part of the solvent, being strongly retained in the liquid and which cannot be evaporated.

FR 3036618 describes NADES, methods in which these NADES are involved, plant extracts obtained by using the NADES and the use of these plant extracts for the preparation of cosmetic compositions.

The NADES are liquid at room temperature and characterized in that they consist of a first and a second compound, each selected from the group comprising the family of polyols, the family of sugars or the family of amino acids and that the first compound and the second compound are selected from different families, the NADES may advantageously further comprise an aqueous phase.

FR 3017292 describes a cosmetic formulation based on vegetable or natural origin, a cosmetic composition containing the formulation, and a process for the preparation of such a cosmetic composition. It also relates to the use of a eutectic solvent as the basis of a cosmetic formulation and to a device comprising a cosmetic composition. The cosmetic formulation base comprises a eutectic solvent consisting of natural or plant molecules. The mixture of a eutectic solvent and of an active ingredient until a homogeneous dispersion is obtained and the resulting homogeneous dispersion is cooled until a balsam texture is obtained.

FR 3046352 describes the cosmetic use of a eutectic solvent consisting of molecules of vegetable or natural origin, as an agent that enhances the barrier function of the epidermis to improve the radiance of the skin, and / or smooth the skin surface and / or smooth the skin and / or improve the complexion of the skin.

The eutectic solvent comprises at least one active ingredient selected from mangiferin, mangustin, rutin, apigenin, esculin, mannitol, Baaleinin lupeol acetate, coumarins, polyols, triterpenes, saponins, carotenoids, polyphenols or a mixture thereof or plant extracts rich in these molecules, such as an ash manna extract (fraxinus ornus) rich in mannitol, an extract of chicle gum (Manilkara zapota) rich in lupeol acetate, an extract of scutellar (Scutellaria baïcalensis), rich in bayalein or an extract of green coffee beans, which are rich in chlorogenic acids.

The following problems need to be improved or resolved.

Active ingredients are normally incorporated into cosmetic products as a solution in water. However, after application, water evaporates quickly and the actives remaining in solution precipitate. Once the actives are precipitated they are no longer bioavailable and display no more activity.

Vitamin C (ascorbic acid) is one of the common active ingredients and a compound with proven biological activity for cosmetic application, however it decomposes in presence of water and air, typically in the span of a few days to a few weeks. This behaviour limits its incorporation to cosmetically acceptable and long-term stable formulas.

Recently there has been extensive studies about the possibilities of achieving better skin properties by ensuring the microbiome of the skin is well balanced. One strategy is to deliver living bacteria which display a positive effect on the microbiome. However, it is a challenge to find a suitable medium where the desired bacteria strain remains alive and in the desired concentration, that means no growth or death.

Another problem seems to be that some compounds needed to ensure the functionality of a cosmetic formulation, such as organic UV-filters and preservatives, can penetrate the skin barrier, leading to undesired biological effects. Their activity is only desired on the external surface of the skin - however there should be little possibilities of decreasing their penetration without modifying their chemical structure.

In addition, long-last moisturization of the skin has always being a cosmetic challenge, specially taking sensory of the final formula into consideration. Water-binding substances such as glycerine, propanediol and urea are commonly used, but their alternatives are still relatively scarce. New substances capable of enhancing the perceived moisturization of the skin are needed in order to expand the formulation possibilities.

All these challenges and problems are solved by the inventive eutectic solvent or by a composition, especially cosmetic composition, comprising such an eutectic solvent which is formed from the mixture of three different components selected from the group consisting of Betaine, Glycerol, Urea, Citric Acid, Malic Acid, Glucose, Fructose, Sorbitol, Sucrose, Xylitol, Proline and Choline Chloride.

The eutectic solvent comprises three components with the preferred molar ratio of the components being chosen in the range from a to b to c, wherein a, b and c are chosen in the range from 1 to 10, especially between 1 to 5, especially 1 to 2.

Preferred eutectic solvents comprises three components with a molar ratio of 1 : 1 : 1 or 2 : 1 : 1.

Preferably the eutectic solvent comprises water in addition in an amount up to 40 %, preferably 20 % by weight based on the total mass of the eutectic solvent and water.

The inventive eutectic solvents or a composition comprising the eutectic solvents shows
- a new way to increase the availability of actives by solving them in new hydrophilic solvents which do not evaporate;
- a surprising new way of delivering sensitive actives, e.g. myriceline or ascorbic acid, in a liquid form with reduced degradation compared to water solution;
- a remarkable boost and long-lasting moisturization when compared to usual cosmetic strategies;
- a new way of blocking sweat glands via the water triggered precipitation of water-insoluble salts dissolved in new solvents;
- a new hydrophilic bacteriostatic solvent, in which gram positive and gram negative bacteria can remain dormant and be reactivate by simple dilution in water;
- a new solvent, suitable for AP actives eg. ACH, Magnesium salts, etc. and boosting its anti-transpirant properties when compared to a water solution;
- a solvent that hinders the penetration of cosmetic compounds which activity is expected on the surface of the skin and dermal absorption is not desired (eg. UV-filters and preservatives)

Surprisingly, these new class of solvents was developed by combining two substances selected from the group of sugars, aminoacids, short organic acids, polyols, betaines, quaternary ammonium salts in precise molar ratio leading to the formation of a liquid phase displaying a melting point lower than the individual compounds. In most of the cases, the new liquids do not crystallize as pure substances does, but rather become amorphous solids upon cooling. Therefor no melting point can be determined experimentally, only a glass transition temperature. But these new solvents display a negligible vapor pressure, therefore they do not evaporate at room temperature and pressure.

The new inventive eutectic solvents allows an improved incorporation of active ingredients into cosmetic compositions. The active ingredient is stabilized and more readily available. Therefore, the amount of active ingredients can be reduced in the composition without of an decrease of activity.

As preferred the following active ingredients could be added to the inventive composition:
Glycyrrhiza Inflata Root Extrakt, Silver Citrat, 4-[(Cyclopentylhydroxyphenylacetyl)oxy]-1,1-dimethyl-piperidiniumbromid (GPIP), Isobutylamido Thiazolyl Resorcinol (Thiamidol), Sodium Ascorbyl Phosphate, Folic acid, Tocopherol, Lipoic acid, Kalium-Methoxysalicylate, Vitamin B6 HCI, Pyrus Malus Stem Extract, Glyceryl Glucoside, short and / or long chain hyaluronic acid, 4-Butyl-Resorcin, Octadecen Dioic Säure, Caffeine, Carnitine, Creatine, Cystine, I-tyrosine, Alpha-Glucosylrutin, Taurin, Vitamin C/Ascorbic acid, Glycyrrhizic acid, Niacinamid, Magnesium-Ascorbylphosphat, Ellagic acid, licorice root extract, sea salt, Isoserinol, Ascorbylglucosid, Undecenoylphenylalanin, Kojic acid, arbutin, Zingeron, Dihydromyricetin, 4-Hexyl-Resorcin, Phenylethyl Resorcinol, Ubiquinone, esp. Q10, Cyanomethylphenyl Menthan Carboxamid, Menthoxypropanediol, Menthan Carboxamid Ethylpyridin, Hydroxyethylurea, Climbazol, Epsilon-Poly-L-Lysine, Laureth-9, Piroctone Olamine, Selenium sulfide, zinc pyrithione as well as deodorant- and/or antiperspirant active ingredients and as well as plant- and/or fruit extracts.

The solubility of actives is higher in inventive eutectic solvents than in water and contrarily to oils, these solvents are hydrophilic and have the same (or even superior) capacity to dissolve actives, like folic acid, Thiamidol or even other molecules like tapioca starch.

Water in contact with these liquids is often in a remarkably bound state due to extensive hydrogen bonding interactions. Some of these liquids integrated into cosmetic formulas lead to an improved moisturization of the skin due to extensive water binding.

Surprisingly, the following advantages based on the eutectic solvents according to the invention, which are explained in more detail below.

The combination of the solid ascorbic acid with the inventive eutectic solvents surprisingly yields a liquid due to the decrease in the melting point. This liquid, stable at room temperature, is water-free and can be incorporated into cosmetic formulas. Furthermore, it was shown that the addition of water up to 40% can be used as a strategy to decrease the viscosity of the liquid without increased degradation of the ascorbic acid. This lead to decreased vitamin C degradation compared to water solutions used nowadays.

Some water-insoluble compounds such as uric acid, calcium lactate and I-tyrosine can be dissolved in higher concentration into the created inventive eutectic solvents. When this composition comes in contact with water from sweat in the pores, the compounds precipitate, thus blocking the sweat flow.

Therefore a new antiperspirant composition is developed based on the eutectic solvents according to the invention.

This invention consists in the identification of water-insoluble salts which solubility would be increased in inventive eutectic solvents.

In a second step, due to dilution caused by sweating, the solubility of the salt would tend to decrease, leading to precipitation and blocks the sweat glands.

As an example, increased solubility of calcium citrate (water-insoluble) in a 3-component NADES was tested. Decrease of solubility as water content increases

0,1% calcium citrate in Glucose/Fructose/Sorbitol by increasing water content (25%, 35%, 50% and 75% of water) shows a decrease of solubility as water content increases.

When one or more of the initial compounds are solid at room temperature, it is possible to trigger its crystallization - and if this solid formation happens preferentially inside the sweat gland pores, anti-perspirant activity is observed.

The efficacy of antitranspirant products depends heavily on the aggregation of antiperspirant actives, like aluminum salts, which can be further improved by the right choice of the solvent, leading to a boost in efficacy. The anti-transpirant efficacy of aluminum salts is surprisingly boosted when dissolved in the inventive solvents.

Also this inventive solvent, when liquid at room temperature, can be used for suspending bacteria. Surprisingly, the bacteria are kept in a dormant and no bacterial growth or death takes place, due to the absence of water, or its low activity when present up to 50% wt.

The combination of three of the inventive compounds lead to a mixture displaying a melting point significantly inferior to the individual compounds and liquid at room temperature. The mechanism behind is related to a strong hydrogen-bonding capacity between the molecules. This strong molecular interaction can happen also with molecules solubilized in the mentioned liquid, leading to a decrease in the liberation and dermal-absorption of the compound during pig-skin penetration test.

It was shown that the liberation of caffeine over time is surprisingly lower in eutectic solvents according to the invention when compared to the water solution. This is believed to be the reason behind the, also surprisingly, its lower dermal absorption.

The same phenomena was studied in a W/O emulsion compared to a Nades-in-oil emulsion, both containing 0.5% caffeine

A similar trend was observed with the use of the active molecule Thiamidol.

Also a test is performed to prove the same trend with benzophenone-3, a model molecule with similarities to actives which penetration is undesired.

The eutectic solvents according to the invention shows a reservoir effect. When dissolved in the aqueous phase, actives tend to lose their bioactivity whenever water evaporates, due to their crystallization. By dissolving the actives of interest in the new eutectic solvents, an increase in the availability is observed, leading to a better dermal absorption.

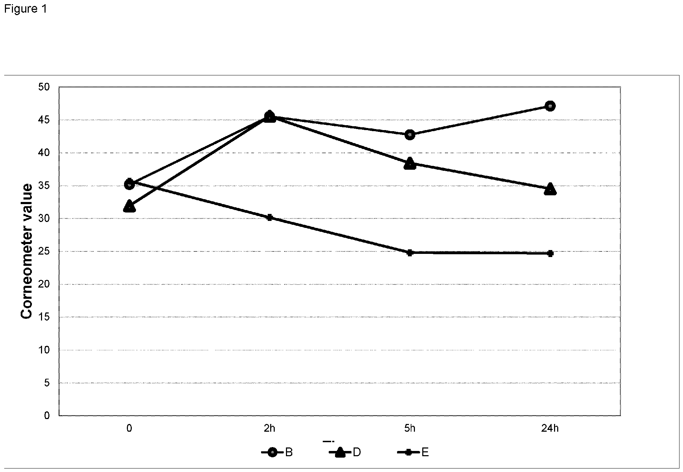

Due to the hydrophilic nature of the new solvents, a remarkable superior in vitro moisturization was measured for an emulsion containing Glucose + Fructose + Sorbitol (1:1:1) when compared to a standard formula (Nivea Soft). The analysis was made in pig skin disks using a corneometer to measure moisture (figure 1).

**Table 1: test preparations (corneometer value, figure 1):**

| | **Sample** | **T = 0** | **T = 2h** | **T = 5h** | **T = 24h** |
|---|---|---|---|---|---|
| B | Glucose + Fructose + Sorbitol (1:1:1) in oil emulsion | 35.2 | 45.5 | 42.7 | 47.1 |
| D | Nivea SOFT | 31.9 | 45.5 | 38.4 | 34.5 |
| E | Control sample | 35.7 | 30.1 | 24.8 | 24.7 |

The eutectic solvents according to the invention (B) shows a long-lasting moisturizing effect (longer than the limit of 24h in vitro) and a positive effect on reducing TEWL (transepidermal water loss).

The inventive compounds were chosen from the group comprising Betaine, Glycerol, Urea, Citric Acid, Malic Acid, Glucose, Fructose, Sorbitol, Sucrose, Xylitol, Proline and Choline Chloride.

Betaine is a quarternary ammonium compound with three methylgroups and structure

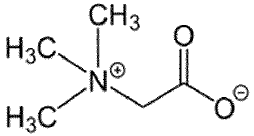

Melting point of betaine is 301 °C.

Choline chloride is an organic compound with the formula (CH3)3NCH2CH2OH]Cl. It is bifunctional, containing both quaternary ammonium salt and an alcohol. The cation is choline, which occurs naturally. It is a white, water soluble salt used mainly in animal feed.

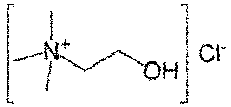

Melting point of choline chloride is 302°C.

Citric acid is a weak organic acid. It occurs naturally in citrus fruits.

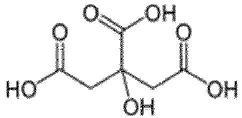

Melting point of citric acid is 156°C.

Glycerol (Glycerin, Propan -1,2,3 -triol) is a common cosmetic ingredient.

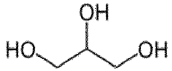

Melting point of glycerol is 18°C.

Malic acid is an organic compound. It is a dicarboxylic acid that is made by all living organisms, contributes to the sour taste of fruits, and is used as a food additive.

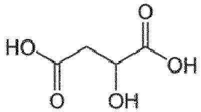

Melting point of malic acid is 130°C.

Urea, chemically the diamide of carbonic acid, is an organic compound with the structure

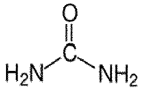

Melting point of urea is 132,5 °C.

Glucose (Glc) is a naturally occurring chemical compound. D-glucose is also referred to as dextrose. D-glucose is the most common monosaccharide.

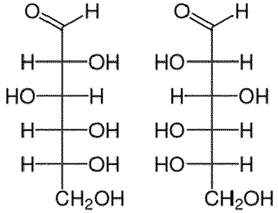

Melting point of glucose is 146°C.

Fructose, or fruit sugar, is a simple ketonic monosaccharide found in many plants,

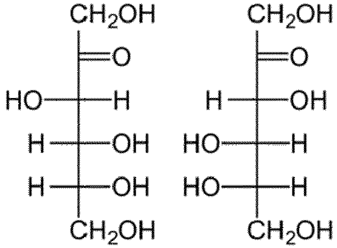

Melting point D, L-Fructose is 129°C.

Sucrose is common sugar. It is a disaccharide, a molecule composed of two monosaccharides: glucose and fructose. Sucrose is produced naturally in plants, from which table sugar is refined. It has the molecular formula C12H22O11.

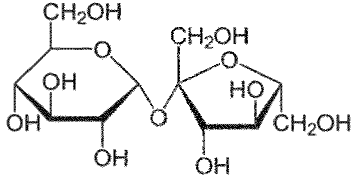

Melting point of Sucrose is 185°C.

Sorbitol (sorbit, also glucitol or hexanehexol) is one of the alditols (sugar alcohols) and is used in many industrially produced foods (food additive E 420) as a sugar substitute, carrier and humectant.

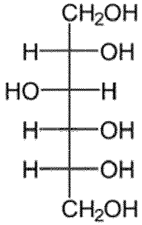

Melting point of Sorbitol is 95°C.

Xylitol is a stereoisomer of the sugar alcohol pentane pentene. As a food additive it bears the name (E 967) and serves as a sugar substitute.

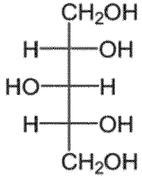

Melting point of xylitol is 94°C.

Proline (L-proline,(S) -pyrrolidine-2-carboxylic acid) is a non-essential proteinogenic heterocyclic secondary α-amino acid.

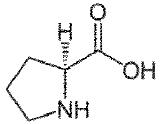

Melting point of proline is 210°C (racemat).

Combinations of three compounds leading to an preferred inventive eutectic solvent are: Glucose + Fructose + Sucrose; Glucose + Xylitol + Fructose; Betaine + Sucrose + Proline; Glucose + Fructose + Sorbitol; Malic acid + Glucose + Fructose; Malic acid + Proline + Choline Chloride; Urea + Glucose + Citric acid; Urea + Glucose + Fructose.

**Table 1: preferred molar ratio of preferred inventive compounds:**

| Comp. A | Comp. B | Comp. C | Mol(A) | Mol(B) | Mol(C) |
|---|---|---|---|---|---|
| Glucose | Sucrose | Fructose | 1 | 1 | 1 |
| Betaine | Sucrose | Proline | 5 | 2 | 2 |
| Glycerin | Sucrose | Proline | 9 | 1 | 4 |
| Glucose | Fructose | Sorbitol | 1 | 1 | 1 |
| Glycerol | Xylitol | Fructose | 2 | 3 | 3 |
| Urea | Glucose | Citric Acid | 1 | 1 | 1 |
| Urea | Glucose | Fructose | 1 | 1 | 1 |

A further example for actives solubilization was made. The inventive solvent comprising Betaine/Sucrose/Proline solubilize Myricelin better than water.

These examples shows the suprising and improved possibilities to incorporate active ingredients into cosmetic compositions.

The inventive composition comprising one or more of an eutectic solvents is preferably a part of a cosmetic composition, especially part of a cosmetic composition based on a water in oil emulsion.

As the comparable test shows, the inventive solvent and composition could be used for improving the incorporation of active ingredients into cosmetic compositions and also for improving the stability of active ingredients in cosmetic compositions and the availability of active ingredients from cosmetic compositions.

The inventive composition could be used for an improved moisturization of the skin.

## Claims

1. An eutectic solvent formed from the mixture of three different components selected from the group consisting of Betaine, Glycerol, Urea, Citric Acid, Malic Acid, Glucose, Fructose, Sorbitol, Sucrose, Xylitol, Proline and Choline Chloride.

2. Solvent according to claim 1 wherein the molar ratio of the three components being chosen in the range from a to b to c, wherein a, b and c are chosen in the range from 1 to 10, especially between 1 to 5, especially 1 to 2.

3. Solvent according to claim1 or 2 wherein the molar ratio of the three components were chosen in the range form 1 : 1 : 1 or 2 : 1 : 1.

4. Eutectic solvent according to one of the clams 1 to 3 **characterized in that** the eutectic solvent is selected from
- Glucose, Fructose and Sucrose;
- Glucose, Xylitol and Fructose;
- Betaine, Sucrose and Proline;
- Glycerol, Sucrose and Proline;
- Glucose, Fructose and Sorbitol;
- Glycerol, Xylitol and Fructose;
- Malic acid, Glucose and Fructose;
- Malic acid, Proline and Choline Chloride;
- Urea, Glucose and Citric acid and/or
- Urea, Glucose and Fructose,

5. Solvent according to one of the claims 1 to 4 **characterized in that** the eutectic solvent is selected from
- Glucose, Sucrose and Fructose with a molar ration of 1 : 1 : 1;
- Betaine, Sucrose and Proline with a molar ration of 5 : 2 : 2;
- Glycerol, Sucrose and Proline with a molar ration of 9: 1 : 4;
- Glucose, Fructose and Sorbitol with a molar ration of 1 : 1 : 1;
- Glycerol, Xylitol and Fructose with a molar ration of 2 : 3 : 3;
- Urea, Glucose and Citric Acid with a molar ration of 1 : 1 : 1 and/or
- Urea, Glucose and Fructose with a molar ration of 1 : 1 : 1.

6. A composition comprising one or more of an eutectic solvent according to one of the claims 1 to 5.

7. Composition according to claim 6 as a cosmetic composition.

8. Composition according to claim 7 wherein the cosmetic composition is based on a water in oil emulsion.

9. Composition according to claim 6, 7 or 8 **characterized in that** the eutectic solvent comprises water in an amount up to 40 percent by weight, preferred up to 20 percent by weight, based on the total mass of the eutectic solvent and water.

10. Composition according to one of the claims 6 to 9 comprising one or more active ingredients, especially cosmetic active ingredients.

11. Composition according to claim 10 wherein the active ingredients were chosen from Glycyrrhiza Inflata Root Extrakt, Silver Citrat, 4-[(Cyclopentylhydroxyphenylacetyl)oxy]-1,1-dimethyl-piperidiniumbromid (GPIP), Isobutylamido Thiazolyl Resorcinol (Thiamidol), Sodium Ascorbyl Phosphate, Folic acid, Tocopherol, Lipoic acid, Kalium-Methoxysalicylate, Vitamin B6 HCI, Pyrus Malus Stem Extract, Glyceryl Glucoside, short and / or long chain hyaluronic acid, 4-Butyl-Resorcin, Octadecen Dioic Säure, Caffeine, Carnitine, Creatine, Cystine, I-tyrosine, Alpha-Glucosylrutin, Taurin, Vitamin C/Ascorbic acid, Glycyrrhizic acid, N-Acetylhydroxyprolin, Niacinamid, Magnesium-Ascorbylphosphat, Ellagic acid, licorice root extract, sea salt, Isoserinol, Ascorbylglucosid, Undecenoylphenylalanin, Kojic acid, arbutin, Zingeron, Dihydromyricetin, 4-Hexyl-Resorcin, Phenylethyl Resorcinol, Ubiquinone, esp. Q10, Cyanomethylphenyl Menthan Carboxamid, Menthoxypropanediol, Menthan Carboxamid Ethylpyridin, Hydroxyethylurea, Climbazol, Epsilon-Poly-L-Lysine, Laureth-9, Piroctone Olamine, Selenium sulfide, zinc pyrithione, deodorant and/or antiperspirant active ingredients and plant- and/or fruit extracts.

12. Use of a solvent according to one of the claims 1 to 5 for improving the incorporation of active ingredients into cosmetic compositions.

13. Use of a solvent according to one of the claims 1 to 5 for improving the stability of active ingredients in cosmetic compositions.

14. Use of a solvent according to one of the claims 1 to 5 for improving the availability of active ingredients from cosmetic compositions.

15. Use of a composition according to one of the claims 6 to 11 for an improved moisturization of the skin.
